# EUROPEAN PATENT APPLICATION

(11) **EP 4 092 420 A1**
(43) Date of publication of application: **23.11.2022**
(21) Application number: 20913848.6
(22) Date of filing: 14.12.2020
(51) Int. Cl.: G01N 35/00, G01N 1/00

(54) **AUTOMATIC ANALYSIS DEVICE**

(30) Priority: 14.01.2020 JP 2020004002
(71) Applicant: Hitachi High-Tech Corporation, Minato-ku Tokyo 105-6409 (JP); Roche Diagnostics GmbH, 68305 Mannheim (DE); F. Hoffmann-La Roche AG, 4070 Basel (CH)
(72) Inventor: MIZUKI Yusuke, Tokyo 105-6409 (JP); NAKAI Marina, Tokyo 105-6409 (JP); SASAKI Shunsuke, Tokyo 105-6409 (JP); IMAI Kenta, Tokyo 105-6409 (JP); SEILER Alexander, 82377 Penzberg (DE); EHRLICH-WEINREICH Gertraud, 82377 Penzberg (DE)
(74) Representative: MERH-IP Matias Erny Reichl Hoffmann Patentanwälte PartG mbB
(86) International application number: PCT/JP2020/046582
(87) International publication number: WO 2021/145118

(57) **Abstract**

An automatic analyzer capable of reducing the time required for a series of maintenance operations is achieved by displaying operation procedures in a maintenance operation in which the user's maintenance work is linked to the device driving so that the maintenance work procedure proceeds in the order of the maintenance work tools.

The automatic analyzer includes a plurality of processing units (an agitation mechanism 16, a reagent dispensing mechanism 17, a mixer 18, an incubator 19, and a specimen dispensing mechanism 22) for processing a specimen, an analysis unit 20 for analyzing a specimen, a display unit 33, and a control unit 31 for controlling the processing unit, the analysis unit 20, and the display unit 33. The control unit 31 causes the display unit 33 to display a plurality of maintenance work tools (23 and 24) used for maintenance work performed by the operator on the plurality of processing units and causes the display unit 33 to display the maintenance work contents of the plurality of processing units that continuously use one of the plurality of maintenance work tools (23 and 24), selected by the operator via the display unit 33 in conjunction with the operation of the processing unit under maintenance.

## Description

### Technical Field

The present invention relates to an automatic analyzer.

### Background Art

For automatic analyzers, there is a known guidance technology for allowing users to correctly operate inspection devices. Inspection devices require complicated maintenance work, and an operation mistake by a user at time of maintenance work results in a malfunction of the device in some cases.

To cope with this, Patent Document 1 discloses a technology that assists user's operations by displaying, on an operation screen, a method of handling a malfunction that has occurred in the device.

### Prior Art Document

### Patent Document

Patent Document 1: JP-2015-092197-A

### Summary of the Invention

### Problem to be Solved by the Invention

According to Patent Document 1, in order to allow a user to perform operations without mistakes when the user handles an inspection device, inspection device operations by the user are assisted by displaying, on a work operation screen, an operation procedure to be followed by the user.

However, there is a problem that assistance by merely displaying on the screen may result in, for a user, an omission in the work in complicated maintenance work.

That is, for a user, enhancement of the workability, suppression of work omissions, and reduction of work time are not taken into consideration about maintenance work for a plurality of maintenance sites with a plurality of maintenance work tools, and further improvements are required.

An object of the present invention is to solve the problem described above and achieve an automatic analyzer capable of reducing the time required for a series of maintenance operations by displaying operation procedures in a maintenance operation in which the user's maintenance work is linked to the device driving so that the maintenance work procedure proceeds in the order of the maintenance work tools.

### Means for Solving the Problem

In order to achieve the object described above, the present invention is configured in the following manner.

An automatic analyzer includes a plurality of processing units for processing a specimen, an analysis unit for analyzing the specimen, a display unit, and a control unit for controlling the processing units, the analysis unit, and the display unit. In the automatic analyzer, the control unit causes the display unit to display a plurality of maintenance work tools used for maintenance work performed by an operator on the plurality of processing units and causes the display unit to display, in conjunction with operations of the processing units for which maintenance is performed, maintenance work contents of the plurality of processing units for which one of the plurality of maintenance work tools selected by the operator via the display unit is continuously used.

### Advantages of the Invention

According to the present invention, an automatic analyzer capable of reducing the time required for a series of maintenance operations can be achieved by displaying operation procedures in a maintenance operation in which the user's maintenance work is linked to the device driving so that the maintenance work procedure proceeds in the order of the maintenance work tools.

### Brief Description of Drawings

[Fig. 1] Fig. 1 is a schematic configuration diagram of an automatic analyzer.
[Fig. 2] Fig. 2 is a schematic side view of the automatic analyzer.
[Fig. 3] Fig. 3 is a flowchart illustrating a guide process of a maintenance work performed by a user of the automatic analyzer.
[Fig. 4] Fig. 4 is a diagram showing an example of a first tool and a second tool.
[Fig. 5] Fig. 5 is a diagram showing the maintenance work sequence guide screen.
[Fig. 6] Fig. 6 is a diagram showing a cleaning tool preparation confirmation screen.
[Fig. 7] Fig. 7 is a diagram showing a maintenance work continuation instruction request screen.
[Fig. 8] Fig. 8 is a diagram showing a guide screen of the cleaning with the first tool.
[Fig. 9] Fig. 9 is a diagram showing a guide screen of the cleaning with the second tool.
[Fig. 10] Fig. 10 is a flowchart illustrating a guide process when cleaning is performed in the order of processing units.
[Fig. 11] Fig. 11 is a diagram showing a site 1 cleaning guide screen 401.
[Fig. 12] Fig. 12 is a diagram showing a site 2 cleaning guide screen.
[Fig. 13] Fig. 13 is a diagram showing a site 3 cleaning guide screen.
[Fig. 14] Fig. 14 is a diagram showing a site 4 cleaning guide screen.

### Modes for Carrying Out the Invention

Embodiments for carrying out the present invention are explained below by using the figures.

### Embodiments

An automatic analyzer in the present embodiment includes: an input unit for receiving an instruction for starting maintenance work performed by a user (operator); and a control unit for performing control such that a predetermined first process is performed according to input through the input unit.

In addition, the automatic analyzer includes: a first processing unit, a second processing unit, and a third processing unit that are arranged spaced apart from each other and perform mutually different processes; and a display unit for displaying information for assisting a user with maintenance work of the first processing unit, the second processing unit, and the third processing unit.

The first processing unit and the second processing unit are processing units that require maintenance work using a first tool (first maintenance work tool).

In addition, the third processing unit is a processing unit that requires maintenance work using a second tool (second maintenance work tool), and the display unit displays information such that the maintenance work of the first processing unit and the maintenance work of the second processing unit are performed continuously.

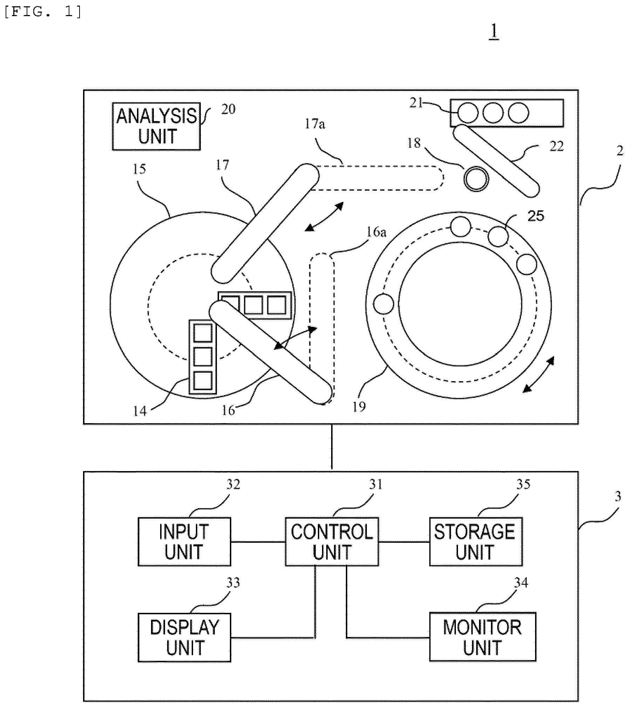

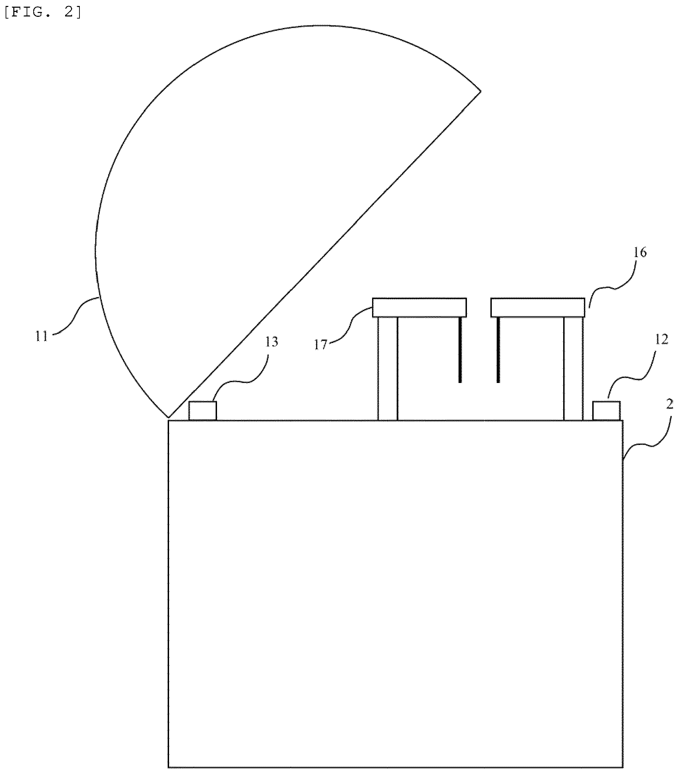

Fig. 1 is a schematic configuration diagram of the automatic analyzer, and shows a plane of an automatic analyzer 1. Fig. 2 is a schematic side view of the automatic analyzer.

In Fig. 1, the automatic analyzer 1 includes a measurement device 2 for performing measurement of specimens, and a control device 3. The measurement device 2 and the control device 3 are connected to each other by a signal line, and a control signal from the control device 3 controls the measurement device 2.

The measurement device 2 includes: a cool box 15 for storing therein reagent vessels 14; an agitation mechanism 16 having an agitating rod for agitating reagents in the reagent vessels 14 stored in the cool box 15; and a reagent dispensing mechanism 17 having a dispensing probe for dispensing the agitated reagents from the reagent vessels 14.

In addition, the measurement device 2 includes: a mixer 18 for agitating, in a vessel, the reagents dispensed by the reagent dispensing mechanism 17; and an incubator 19 for keeping the temperature of reaction vessels 25 after the agitation.

In addition, the measurement device 2 includes: an analysis unit 20 for analyzing a specimen; and a specimen dispensing mechanism 22 having a dispensing probe for dispensing the specimen contained in a specimen vessel 21 to a reaction distant-view vessel 25 arranged in the incubator 19.

The cool box 15, the agitation mechanism 16, the reagent dispensing mechanism 17, and the incubator 19 configure drive units of the automatic analyzer 1, and are each drive-controlled by the control device 3.

Each drive unit includes a motor and a position sensor, and the position sensor allows a decision whether or not the drive unit is at a predetermined position. In addition, the agitation mechanism 16 and the reagent dispensing mechanism 17 can move to retraction positions 16a and 17a, respectively, represented by dotted lines when they are not necessary.

The control device 3 has: a control unit 31 for controlling a process and an operation performed by each constituent site of the automatic analyzer 1; an input unit 32 for receiving a work request from a user (operator); a display unit 33 for outputting an operation instruction to the user; a monitor unit 34 for monitoring the state of the measurement device 2; and a storage unit 35 for storing a process performed by the control unit 31. The monitor unit 34 monitors the state of the measurement device 2 by using position sensor information and the like, and monitors whether or not the measurement device 2 is performing measurement of a specimen.

The control unit 31 of the control device 3 can be configured by using a central processing unit (CPU). The control device 3 can be entirely configured by using a personal computer (PC) including the control unit 31 including a CPU, the input unit 32 such as a keyboard or a mouse, the display unit 33 such as a liquid crystal display, the storage unit 35 such as a semiconductor memory, and the like. In that case, the monitor unit 34 that monitors the position sensor information and the like may be achieved by program execution by the CPU which is the control unit 31.

As shown in Fig. 2, the measurement device 2 has: a device cover 11 for covering drive units of the measurement device 2 such that a user is prevented from touching the drive units; a locking mechanism 12 of the device cover 11; and an opening and closing sensor 13 of the device cover 11. The automatic analyzer includes an interlock mechanism (not shown) for performing interlock control of turning off the motors of the drive units (stopping operations of processing units) when the opening and closing sensor 13 has sensed that the device cover 11 is open, such that the device is prevented from being driven unexpectedly while the user is performing work in a state in which the device cover 11 is opened (open).

This interlock mechanism ensures safety of user's work. Such an interlock mechanism can be achieved by a program executed by the control unit 31 of the control device 3, and the opening and closing sensor 13 and the locking mechanism 12 also constitute part of the interlock mechanism.

That is, the control unit 31 performs the interlock control of locking the locking mechanism 12 such that the user is prevented from accessing the measurement device 2 while the automatic analyzer 1 is being driven, and unlocking the locking mechanism 12 such that the user is allowed to access the measurement device 2 after drive completion.

The plurality of processing units mentioned above include the mixer 18, the incubator 19, the reagent dispensing mechanism 17, the specimen dispensing mechanism 22, and the agitation mechanism 16.

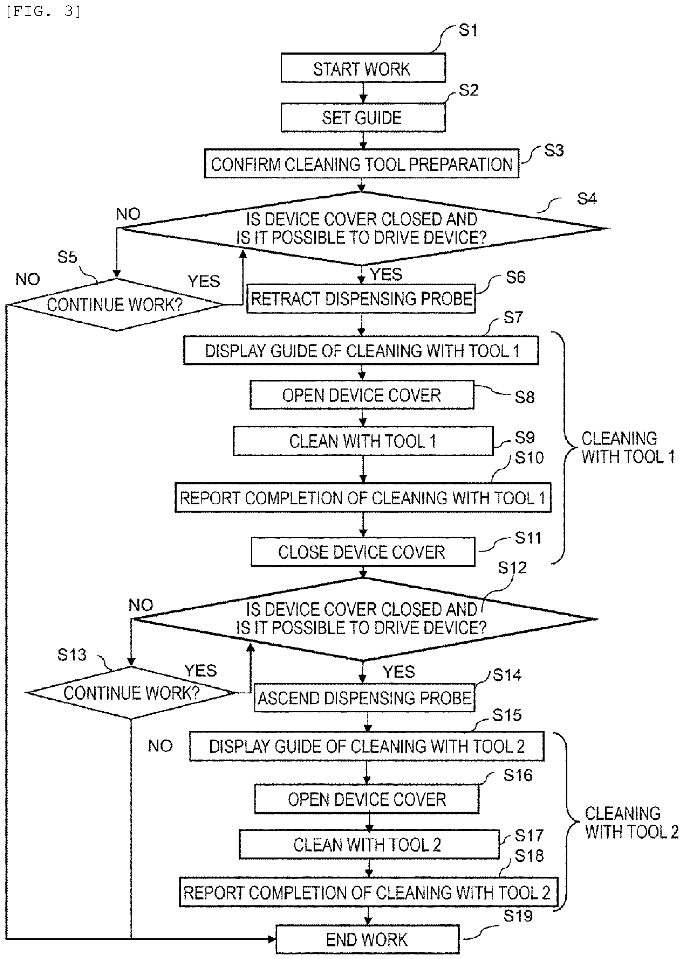

Fig. 3 is a flowchart illustrating a guide process of a maintenance work performed by a user of the automatic analyzer 1. The guide process includes: a dispensing probe retracting process (retraction operation) in which a display operation of the display unit 33 is controlled under the control of the control unit 31, and the automatic analyzer 1 is driven for preparations for user's work; a process of the maintenance work performed by the user using the first tool; a dispensing probe ascending process for preparations for user's work similarly to the dispensing probe retracting process; and a process of the maintenance work performed by the user using the second tool.

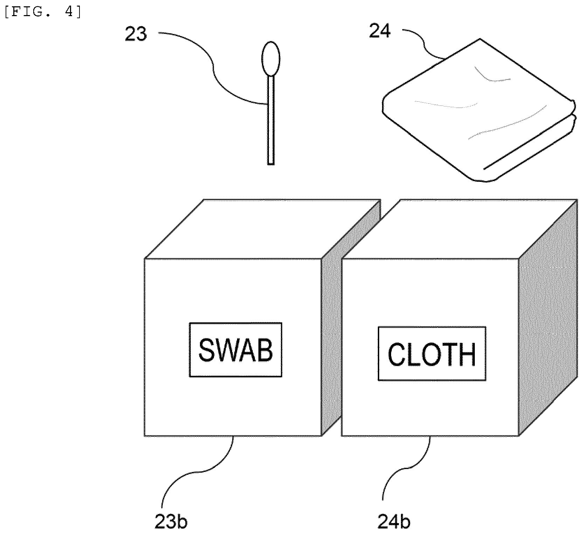

Fig. 4 is a diagram showing an example of the first tool and the second tool. As shown in Fig. 4, this is an example in which swabs 23 and cloth 24 are the first tool and the second tool. Which of the swabs 23 and the cloth 24 is set as the first tool or the second tool can be decided as desired by a user. The swabs 23 are contained in a swab storage container 23b, and the cloth 24 is contained in a cloth storage container 24b. The swab storage container 23b and the cloth storage container 24b are arranged at any positions near the automatic analyzer 1 as desired by the user.

Note that before the dispensing probe retracting process mentioned later, the user may be allowed to select the maintenance order of the automatic analyzer 1 between the order of tools to be used and the processing unit order. In addition, a step may be provided in which a name of a required tool is displayed in advance for prompting the user to check whether the tool has been prepared.

At the dispensing probe retracting process of Step S6 performed after execution of Steps S1 to S5 mentioned later, the dispensing probe of the reagent dispensing mechanism 17 and the dispensing probe of the specimen dispensing mechanism 22 are stored inside the automatic analyzer 1 on the basis of a predetermined drive program. Thereby, three-dimensional hindrances on the automatic analyzer 1 are reduced, and, in this state, the user can easily access each processing unit that requires maintenance.

After the dispensing probe retracting process of Step S6 is completed, the procedure proceeds to Step S7, and a guide A (mentioned later) which is a guide for cleaning (maintenance work) with the first tool is displayed on the monitor unit 34. The guide A includes a message prompting the user to perform maintenance of two or more different processing units continuously by using one type of tool.

After the cleaning with the first tool is completed, the dispensing probe ascending process is executed on the basis of a completion report from the user. In the dispensing probe ascending process, on the basis of a predetermined drive program, the dispensing probe of the reagent dispensing mechanism 17 and the dispensing probe of the specimen dispensing mechanism 22 which have been stored inside the automatic analyzer 1 are ascended in the order opposite to the dispensing probe retracting process. This process is required when the dispensing probes are included in portions to be cleaned next, and may not be implemented when the dispensing probes are not included in the portions to be cleaned subsequently.

After the dispensing probe ascending process is completed, and Steps S8 to S14 mentioned later are executed, a guide B (mentioned later) which is a guide for cleaning with the second tool is displayed on the monitor unit 34 at Step S15. The guide B includes a message prompting the user to perform maintenance of a processing unit different from the processing units displayed on the guide A by using a tool different from the first tool.

After the cleaning with the second tool is completed, the maintenance work is completed on the basis of a report from the user.

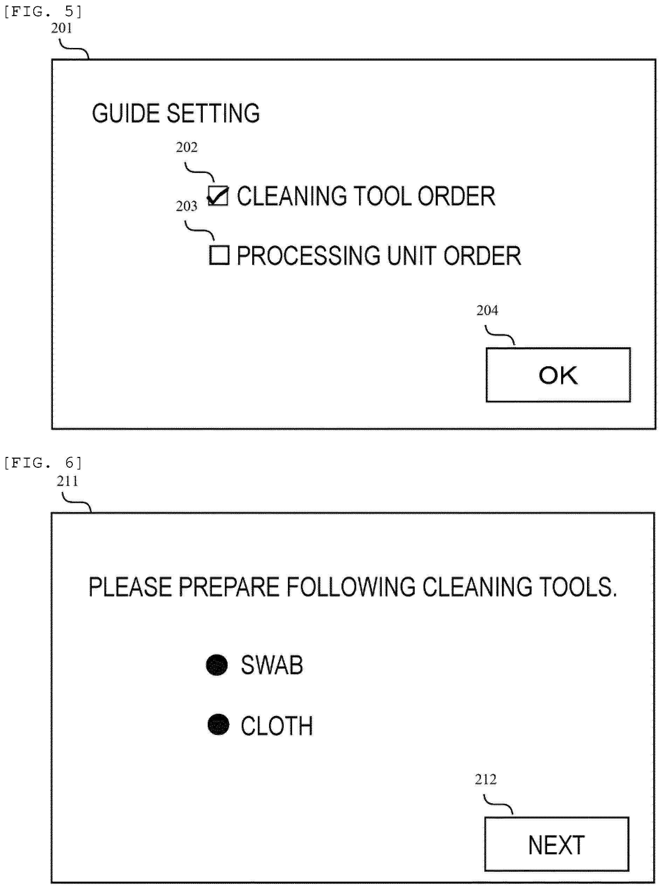

Fig. 5 to Fig. 9 are guide display examples at time of the maintenance work for the automatic analyzer 1. As shown in these figures, the display unit 33 doubles as the input unit 32, and various types of instruction and report can be input by touching a predetermined position on the screen. Alternatively, various types of instruction and report can be input also by clicking a predetermined position on the screen using an input device such as a mouse. A first embodiment is explained more specifically by using these guide display examples at time of the maintenance work.

At Step S1 in Fig. 3, when a user requests maintenance work through the input unit 32, the work is started. Then, the procedure proceeds to Step S2 which is guide setting, and the display unit 33 displays a maintenance work sequence guide screen 201 as shown in Fig. 5.

The maintenance work sequence guide screen 201 is a screen for the user (operator) to select whether to perform the maintenance work in the order of a plurality of cleaning tools (maintenance work tool order) or in the order of a plurality of processing units.

The maintenance work sequence guide screen 201 includes a message related to orders of proceeding of the maintenance work, and the user selects either the cleaning tool order (maintenance work tool order) or the processing unit order displayed on the maintenance work sequence guide screen 201. Since it is considered that there are some users who prefer the processing unit order depending on experience the users have had, a highly flexible functionality can be provided by providing this guide. When the user is to enable a selection box of the cleaning tool order 202 between the selection box of the cleaning tool order 202 and a selection box of the processing unit order 203, the user checks the selection box of the cleaning tool order 202 as shown in the figure, and then gives an instruction about the procedure of the maintenance work by a maintenance work procedure instruction unit 204.

Next, at Step S3, the display unit 33 displays a cleaning tool preparation confirmation screen 211 as shown in Fig. 6. On the cleaning tool preparation confirmation screen 211, the names of all tools (the swabs 23 and the cloth 24 in the example shown in the figure) to be used in a series of maintenance work are displayed on one screen of the display unit 33, and the user is prompted to check whether there is an omission of the preparation of a required tool. The user follows this guide, prepares each tool via the display unit 33, and then gives an instruction for the start of the maintenance work by a maintenance work start instruction unit 212 by a tool preparation completion report. Due to the existence of the guide, it becomes possible for the user to prepare the tools efficiently without omissions.

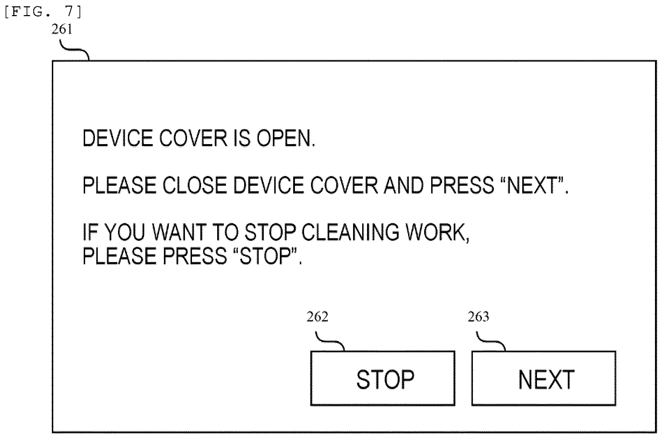

Next, at Step S4, the control unit 31 requests the monitor unit 34 to monitor the state of the measurement device 2. At this time, when it is determined that the device cover 11 is open on the basis of position sensor information or the like, and it is not possible to implement the maintenance work, the interlock control is performed, a guide by a maintenance work continuation instruction request screen 261 shown in Fig. 7 is displayed on the display unit 33, and the procedure waits for an instruction from the user as to whether to or not to continue the maintenance work. The guide by the maintenance work continuation instruction request screen 261 is displayed to inform the user (operator) that the device cover 11 is open, and allow the user to determine whether to or not to close the device cover 11 and proceed to the next maintenance work step.

When the maintenance work is to be continued, the user closes the device cover 11, and then gives an instruction for continuation by a maintenance work continuation instruction unit 263. The control unit 31 requests the monitor unit 34 to monitor the state of the measurement device 2 again on the basis of the maintenance work continuation instruction.

On the other hand, when the maintenance work is to be stopped, the user gives a maintenance work stop instruction by a maintenance work stop instruction unit 262. Then, the automatic analyzer 1 cancels the work request on the basis of the maintenance work stop instruction at Step S5, and ends the work (Step S19).

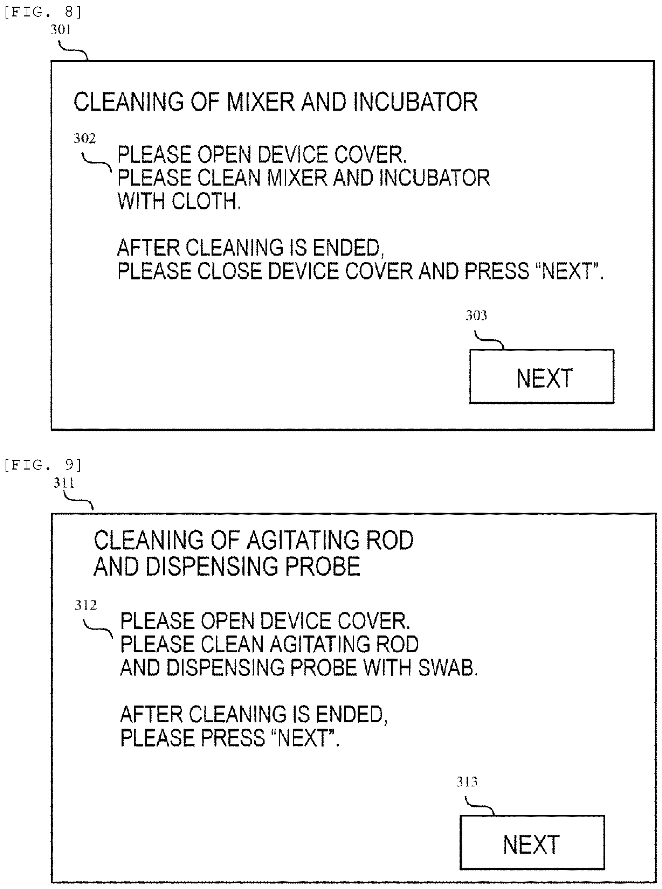

When the device cover 11 is closed, and additionally it is possible to operate drive units, at Step S6, the dispensing probe of the reagent dispensing mechanism 17, which is a drive unit, makes a turn-move and lowers to the retraction position 17a, the dispensing probe of the specimen dispensing mechanism 22, which is a drive unit, makes a turn-move and lowers to a retraction position (not shown), and the agitating rod of the agitation mechanism 16, which is a drive unit, makes a turn-move and lowers to the retraction position 16a. Then, the dispensing probes described above, and the agitating rod of the agitation mechanism 16 are stored inside the automatic analyzer 1. After the drive completion, at Step S7, the control unit 31 sends an unlocking instruction to the locking mechanism 12, and causes the display unit 33 to display a guide screen 301 of the cleaning with the first tool shown in Fig. 8.

On the basis of a guide unit 302 for maintenance work with the first tool shown in Fig. 8, the user first opens the device cover 11 in order to access a portion requiring maintenance (Step S8). Next, at Step S9, the user holds a piece of the cloth 24, which is the first tool, in her/his hand, and wipes off the surface of the mixer 18. Thereafter, while holding the piece of the cloth 24 which is the same as the one used for the cleaning of the mixer 18 in her/his hand, the user continues wiping off the surface of the incubator 19. The maintenance work in this case is work performed by the user (maintenance worker) by using the piece of the cloth 24 for the mixer 18 and the incubator 19.

After the maintenance work is ended, in order to report completion of the cleaning with the first tool (Step S10), the user closes the device cover 11 (Step S11). Thereafter, a report instruction from a first tool maintenance work completion report unit 303 is sent to the input unit 32 by the display unit 33. The control unit 31 requests the monitor unit 34 to monitor the state of the measurement device 2. At this time, when it is determined that the cover 11 of the automatic analyzer 1 is open on the basis of position sensor information or the like, and it is not possible to implement the maintenance work, the guide 261 shown in Fig. 7 is displayed on the display unit 33, and the procedure waits for an instruction from the user as to whether to or not to continue the maintenance work (Step S12). When the maintenance work is to be continued, the user closes the cover 11, and then gives an instruction for continuation by the maintenance work continuation instruction unit 263 (Step S13). The control unit 31 requests the monitor unit 34 to monitor the state of the measurement device 2 again on the basis of the maintenance work continuation instruction. On the other hand, when the maintenance work is to be stopped, the user gives a stop instruction by the maintenance work stop instruction unit 262. The automatic analyzer 1 cancels the work request on the basis of the maintenance work stop instruction (Step S19).

When the device cover 11 is closed, and additionally it is possible to operate drive units of the automatic analyzer 1, the dispensing probes of the reagent dispensing mechanism 17 and the specimen dispensing mechanism 2, and the agitating rod of the agitation mechanism 16 are ascended vertically, and each of the tips is stopped at a height at which it is sufficiently exposed out of the inside of the automatic analyzer 1 (Step S14). Thereby, it becomes possible for the user to access the site cleaning portion. After the drive completion, the display unit 33 displays a guide screen 311 of the cleaning with the second tool shown in Fig. 9 as the next procedure (Step S15).

On the basis of a guide unit 312 for the maintenance work with the second tool on the guide screen 311 of the cleaning with the second tool shown in Fig. 9, the user opens the device cover 11 (Step S16), switches to a swab 23, which is the second tool, from the piece of the cloth 24, which is the first tool, and wipes off the surface of the agitating rod of the agitation mechanism 16. Thereafter, while holding the swab 23 which is the same as the one used for the cleaning of the agitating rod of the agitation mechanism 16 in her/his hand, the user continues wiping off the surface of the dispensing probe of the reagent dispensing mechanism 17 (Step S17).

After the maintenance work is ended, the user sends a completion report to the input unit 32 by a second tool maintenance work completion report unit 313, and the entire maintenance work is ended (Steps S18 and S19).

As the guide screen 301 of the cleaning with the first tool and the guide screen 311 of the cleaning with the second tool shown in Fig. 8 and Fig. 9, maintenance work contents of a plurality of processing units for which one tool, the first tool or the second tool, is used continuously are displayed on one screen displayed by the display unit 33.

With the configuration and operations of the automatic analyzer 1 mentioned in detail thus far, by linking user's work to device driving, setting the maintenance work to be performed in the cleaning tool order, and guiding a user by displaying on the display unit 33 such that processing units that require use of the same cleaning tool are maintained consecutively, an automatic analyzer that can simplify user's operation even in complicated maintenance work can be provided.

That is, an automatic analyzer capable of reducing the time required for a series of maintenance operations can be achieved by displaying operation procedures in a maintenance operation in which the user's maintenance work is linked to the device driving so that the maintenance work procedure proceeds in the order of the maintenance work tools.

Next, for comparison of superiority of the first embodiment, a specific procedure of an example of a case where cleaning is performed not in the tool order, but in the processing unit order is explained.

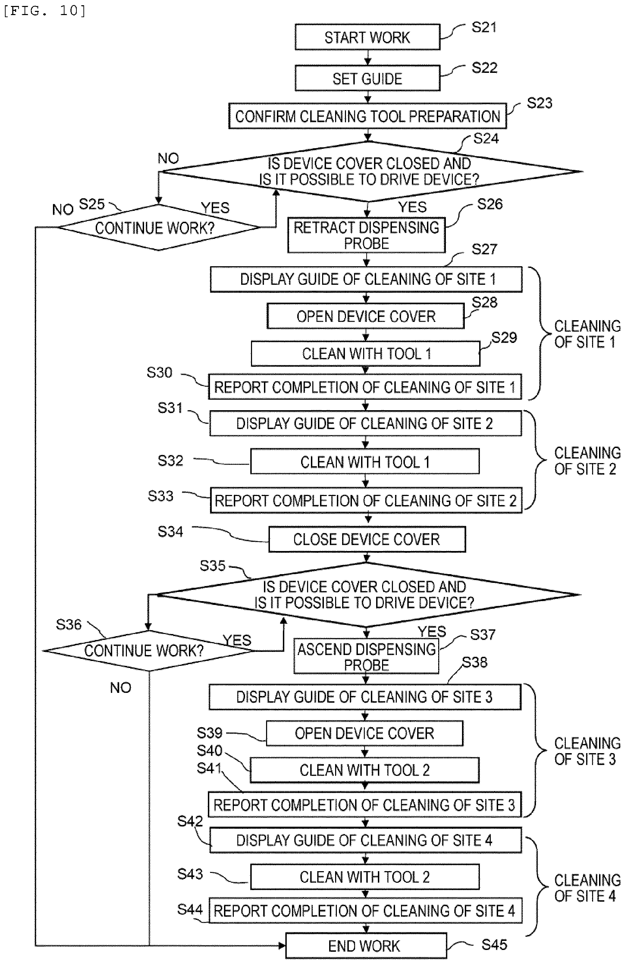

Fig. 10 is a flowchart illustrating a guide process when cleaning is performed in the order of processing units. At this time, the guide process includes: a dispensing probe retracting process in which the automatic analyzer 1 is driven under the control of the control unit 31, and preparations are made for work by a user; a process in which the user performs maintenance work of the first site (processing unit); a process in which the user performs maintenance work for the second site (processing unit); a dispensing probe ascending process in which preparations are made for work by the user similarly to the dispensing probe retracting process; a process in which the user performs maintenance work for the third site (processing unit); a process in which the user performs maintenance work for the fourth site (processing unit); and a process in which the user performs maintenance work by using the first and second tools.

The specific procedure of the process shown in Fig. 10 is explained by comparison with the case where cleaning is performed in the tool order, and extracting differences therefrom.

On the maintenance work sequence guide screen 201 shown in Fig. 5, the user enables the selection box of the processing unit order 203, and then gives an instruction for the procedure of the maintenance work by the maintenance work procedure instruction unit 204.

The control unit 31 requests the monitor unit 34 to monitor the state of the measurement device 2. At this time, when it is determined that the device cover 11 is open on the basis of position sensor information or the like, and it is not possible to implement the maintenance work, the guide 261 shown in Fig. 7 is displayed on the display unit 33, and the procedure waits for an instruction from the user as to whether to or not to continue the maintenance work. When the maintenance work is to be continued, the user closes the device cover 11, and then gives an instruction for continuation by the maintenance work continuation instruction unit 263. The control unit 31 requests the monitor unit 34 to monitor the state of the measurement device 2 again on the basis of the maintenance work continuation instruction. On the other hand, when the maintenance work is to be stopped, the user gives a stop instruction by the maintenance work stop instruction unit 262. The automatic analyzer 1 cancels the work request on the basis of the maintenance work stop instruction.

When it is possible to operate drive units of the automatic analyzer 1, the dispensing probes of the reagent dispensing mechanism 17 and the specimen dispensing mechanism 22, which are drive units, make turn-moves and lower to the retraction position 17a and the like, the agitating rod of the agitation mechanism 16 makes a turn-move and lowers to the retraction position 16a, and the dispensing probes and the agitating rod are stored inside the automatic analyzer 1 (Steps S21 to S26).

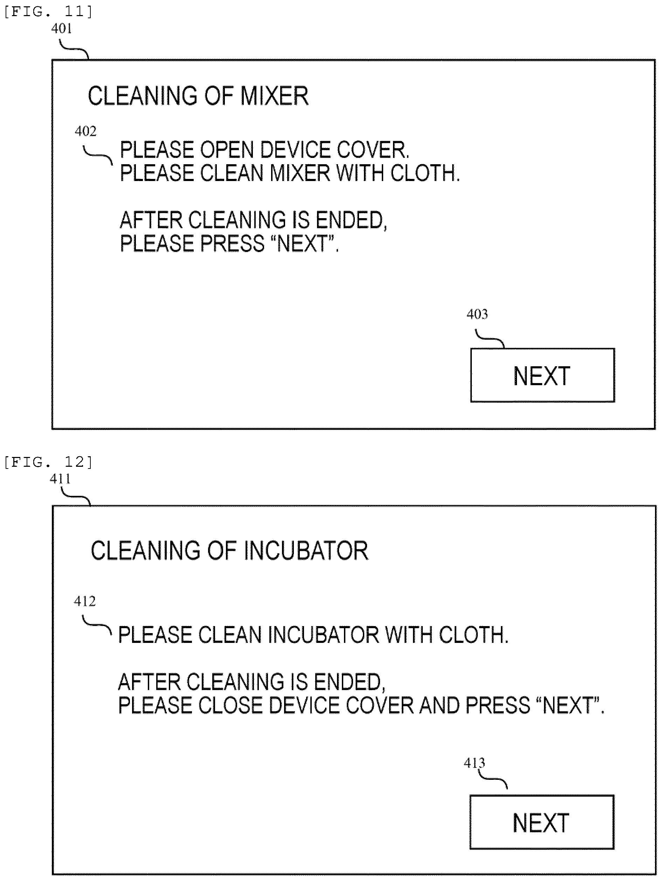

After completion of the retraction of the dispensing probes and the agitating rod of the agitation mechanism 16, the control unit 31 sends an unlocking instruction to the locking mechanism 12, and causes the display unit 33 to display a site 1 cleaning guide screen 401 shown in Fig. 11 (Step S27).

On the basis of a maintenance work guide unit 402 for the site 1 shown in Fig. 11, the user first opens the device cover 11 in order to access a portion requiring maintenance (Step S28). Next, the user holds a piece of the cloth 24, which is the first tool, in her/his hand, and wipes off the surface of the mixer 18 (Step S29). After the work is completed, on the basis of a site 1 work completion report 403 from the user (Step S30), the display unit 33 displays a site 2 cleaning guide screen 411 shown in Fig. 12 (Step S31) .

On the basis of a maintenance work guide unit 412 for the site 2 shown in Fig. 12, the user holds a piece of the cloth 24, which is the first tool, again, and wipes off the surface of the incubator 19 (Step S32).

After the maintenance work is ended, the user closes the device cover 11, and then a work completion report is sent to the input unit 32 by a site 2 work completion report unit 413 from the display unit 33 (Steps S33 and S34). The control unit 31 requests the monitor unit 34 to monitor the state of the measurement device 2. At this time, when it is determined that the device cover 11 is open on the basis of position sensor information or the like, and it is not possible to implement the maintenance work, the guide 261 shown in Fig. 7 is displayed on the display unit 33, and the procedure waits for an instruction from the user as to whether to or not to continue the maintenance work (Steps S35 and S36).

When the maintenance work is to be continued, the user closes the device cover 1, and then gives an instruction for continuation by the maintenance work continuation instruction unit 263. The control unit 31 requests the monitor unit 34 to monitor the state of the measurement device 2 again on the basis of the maintenance work continuation instruction.

On the other hand, when the maintenance work is to be stopped, the user gives a stop instruction by the maintenance work stop instruction unit 262. The automatic analyzer 1 cancels the work request on the basis of the maintenance work stop instruction (Steps S36 and S45).

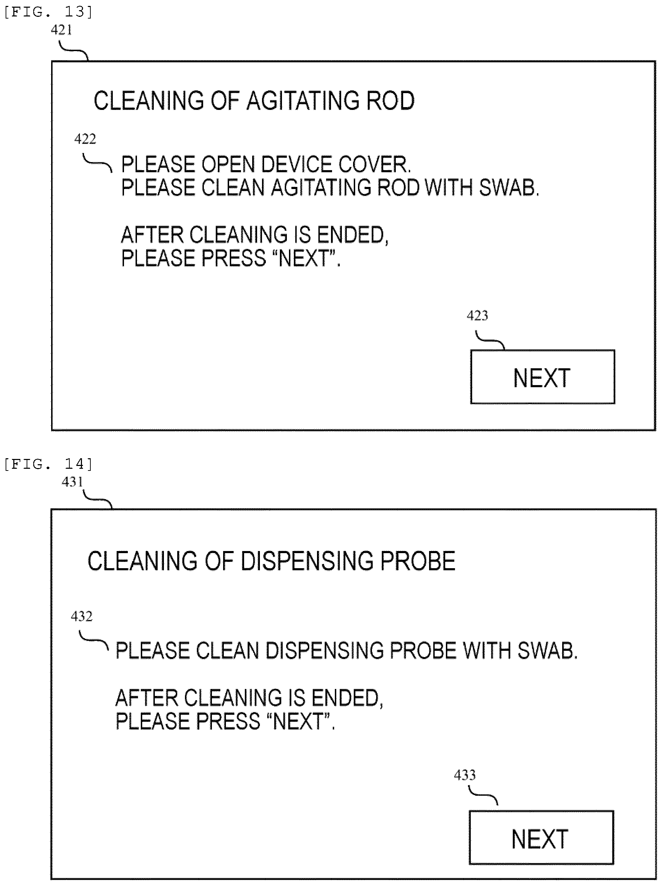

At Step S35, when it is possible to operate drive units of the automatic analyzer 1, the dispensing probes of the reagent dispensing mechanism 17 and the specimen dispensing mechanism 22, and the agitating rod of the agitation mechanism 16 are ascended vertically, and each of the tips is stopped at a height at which it is sufficiently exposed out of the inside of the automatic analyzer 1 (Step S37). Thereby, it becomes possible for the user to access the site cleaning portion. After the drive completion, the display unit 33 displays a guide screen 421 shown in Fig. 13 as the next procedure (Step S38).

On the basis of a maintenance work guide unit 422 for the site 3 on a site 3 cleaning guide screen 421 shown in Fig. 13, the user opens the device cover 11, switches to a swab 23, which is the second tool, from the piece of the cloth 24, which is the first tool, and wipes off the surface of the agitating rod of the agitation mechanism 16 (Steps S39 and S40). After the work is completed, on the basis of an instruction on a site 3 work completion report unit 423 from the user, the display unit 33 displays a site 4 cleaning guide screen 431 shown in Fig. 14 (Steps S41 and S42) .

On the basis of a maintenance work guide unit 432 for the site 4 shown in Fig. 14, the user holds a swab 23, which is the second tool, again, and wipes off the surface of the incubator 19 (Step S43) .

After the maintenance work is ended, the user sends a work completion report to the input unit 32 by a site 4 work completion report unit 433, and the entire maintenance work is ended (Steps S44 and S45) .

If the contents of the process shown in Fig. 10 are compared with the contents of the process shown in Fig. 3 in which work is performed consecutively for each tool to be used, the procedure in which work is performed for each site makes it necessary for the user to proceed with the work while checking contents of guide display displayed at each time with the manual work being stopped temporarily for each site to follow the guide display displayed at each time.

In addition, even if work is different merely in the site as a target of the work and can be regarded as the same work in terms of the contents for a user, the work is treated as completely different work by the automatic analyzer 1. Accordingly, as shown in Fig. 11 and Fig. 12, and Fig. 13 and Fig. 14, it is necessary for the user to make a completion report to the automatic analyzer 1 every time the similar work is ended.

Even if the time required for the confirmation and the work completion report is estimated to be long, it takes approximately several seconds, and time loss of each step singly is not so significant.

However, in the procedure mentioned in detail earlier in which work is performed for each tool, since one tool is commonly used for a plurality of sites, it is possible to omit not a single step, but a plurality of steps (cleaning guide display and a cleaning completion report for each site) collectively. In addition to a reduction of the time required for the entire work, the number of times required by the automatic analyzer 1 for confirmations and completion reports decreases, and thus there are advantages that a user is less likely to feel cumbersomeness and that the work efficiency can be enhanced.

Note that whereas the operation input unit 32 and the display unit 3 at which display of work, a completion report, and so on are made are included in the control device 3 in the configuration in the examples mentioned above, it is also possible, for example, to use a discrete device like a tablet including the input unit 32 and the display unit 33.

In addition, whereas the swabs 23 and the cloth 24 are two examples of maintenance work tools in the examples mentioned above, the present invention can also be applied to a case where three or more maintenance work tools are used.

In addition, the automatic analyzer according to the present invention can be applied to both immunoassay automatic analyzers and biochemical analysis automatic analyzers.

In addition, the reagent dispensing mechanism 17 and the specimen dispensing mechanism can be collectively referred to as a dispensing mechanism.

### Reference Signs List

- 1:: automatic analyzer
- 2:: measurement device
- 3:: control device
- 11:: device cover
- 12:: locking mechanism
- 13:: opening and closing sensor
- 14:: reagent vessel
- 15:: cool box
- 16:: agitation mechanism
- 17:: reagent dispensing mechanism
- 18:: mixer
- 19:: incubator
- 20:: analysis unit
- 21:: specimen vessel
- 22:: specimen dispensing mechanism
- 23:: swab
- 23b:: swab storage container
- 24:: cloth
- 24b:: cloth storage container
- 31:: control unit
- 32:: input unit
- 33:: display unit
- 34:: monitor unit
- 35:: storage unit
- 201: maintenance work sequence guide screen
- 202:: selection box of cleaning tool order
- 203:: selection box of processing unit order
- 204:: maintenance operation procedure instruction unit
- 211:: cleaning tool preparation confirmation screen
- 212:: maintenance work start instruction unit
- 261:: maintenance work continuation instruction request screen
- 262:: maintenance work stop instruction unit
- 263:: maintenance work continuation instruction unit
- 301:: guide screen of cleaning with first tool
- 302:: guide unit for maintenance work with first tool
- 303:: first tool maintenance work completion report unit
- 311:: guide screen of cleaning with second tool
- 312:: guide unit for maintenance work with second tool
- 313:: second tool maintenance work completion report unit
- 401:: site 1 cleaning guide screen
- 402:: maintenance work guide unit for site 1
- 403:: site 1 work completion report unit
- 411:: site 2 cleaning guide screen
- 412:: maintenance work guide unit for site 2
- 413:: site 2 work completion report unit
- 421:: site 3 cleaning guide screen
- 422:: maintenance work guide unit for site 3
- 423:: site 3 work completion report unit
- 431:: site 4 cleaning guide screen
- 432:: maintenance work guide unit for site 4
- 433:: site 4 work completion report unit

## Claims

1. An automatic analyzer comprising:
a plurality of processing units that require maintenance work using a plurality of maintenance work tools,
an analysis unit for analyzing a specimen,
a display unit, and
a control unit for controlling the plurality of processing units, the analysis unit, and the display unit, wherein
the control unit allows the operator to link the maintenance work contents of the plurality of processing units that continuously use at least one of the plurality of maintenance work tools with the operation of the processing unit where maintenance is performed, and displays the operation on the display unit.

2. The automatic analyzer according to claim 1, wherein
the control unit causes the display unit to display the plurality of maintenance work tools used for the maintenance work performed by the operator on the plurality of processing units in conjunction with the maintenance work content.

3. The automatic analyzer according to claim 1, wherein
at least one of the plurality of maintenance work tools is a tool for cleaning the plurality of processing units.

4. The automatic analyzer according to claim 1, wherein
at least one of the plurality of processing units is a dispensing mechanism including a dispensing probe for dispensing a specimen or a reagent.

5. The automatic analyzer according to claim 4, wherein
the operation of the processing unit is at least a retraction operation of the dispensing probe.

6. The automatic analyzer according to claim 1, wherein
a device cover for covering the processing unit is provided, and the control unit performs an interlock control that stops the operation of the processing unit when the device cover is open.

7. The automatic analyzer according to claim 1, wherein
the control unit displays, on one screen of the display unit, work contents of the plurality of processing units that continuously use selected one of the plurality of maintenance work tools.

8. The automatic analyzer according to claim 1, wherein
the control unit displays the plurality of maintenance work tools used for the maintenance work on one screen of the display unit.

9. The automatic analyzer according to claim 6, wherein
when the device cover is open, the control unit causes the display unit to display a display for the operator to determine whether to proceed to the next maintenance work.

10. The automatic analyzer according to claim 1, wherein
the control unit causes the display unit to display a maintenance work sequence guide screen for the operator to select whether to perform the maintenance work in the order of the plurality of maintenance work tools or to perform the maintenance work in the order of the plurality of processing units.

11. The automatic analyzer according to claim 1, wherein
the plurality of processing units include a mixer for agitating the reagent, and an incubator for keeping the temperature of the agitated reagent, one of the plurality of maintenance work tools is a piece of cloth, and the maintenance work is work performed on the mixer and the incubator by the operator using the cloth.

12. The automatic analyzer according to claim 1, wherein
the plurality of processing units includes a dispensing mechanism including a dispensing probe, and an agitation mechanism including an agitating rod and an agitating rod for agitating the reagent,
one of the plurality of maintenance work tools is swab, and
the maintenance work is work performed on the dispensing probe and the agitating rod by the operator using the swab.
